# EUROPEAN PATENT SPECIFICATION

(11) **EP 0 975 648 B1**
(45) Date of publication and mention of the grant of the patent: **04.06.2003**
(21) Application number: 98917345.5
(22) Date of filing: 14.04.1998
(51) Int. Cl.: C07H 15/24, A61K 31/70

(54) **MORPHINE DERIVATIVES**
MORPHIN-DERIVATE
DERIVES DE MORPHINE

(30) Priority: 14.04.1997 US 833203; 07.10.1997 GB 9721137
(43) Date of publication of application: 02.02.2000
(73) Proprietor: UFC LIMITED, Manchester M15 6SY (GB)
(72) Inventor: SCHEINMANN, Feodor, Sale, Cheshire M33 4PW (GB); STACHULSKI, Andrew, Valentine, Swinton, Lancashire M27 5PB (GB); JOEL, Simon, St.Bartholomews, London EC1A 7BE (GB)
(74) Representative: Goodwin, Mark
(86) International application number: GB9801071
(87) International publication number: WO98046618

(56) References cited:
- WO-A-93/03051
- WO-A-95/16050
- WO-A-97/21416
- DE-A- 4 403 709
- L.J.MURPHEY ET AL.: "A Stereospecific Microassay for the Determination of orphine-6-B-D-Glucuronide and Other Active Morphine Metabolites in the Neonatal Guinea PIg." JOURNAL OF LIQUID CHROMATOGRAPHY, vol. 16, no. 12, 1993, pages 2545-2561, XP002076323
- M.W.H.COUGHTRIE ET AL.: "The Enantioselective Glucuronidation of Morphine in Rats and Humans. Evidence for the Involvement of more than one UDP-Glucuronosyl Transferase Isoenzyme." BIOCHEMICAL PHARMACOLOGY, vol. 38, no. 19, 1989, pages 3273-3280, XP002076324
- K.OGURI ET AL.: "Synthesis and Analgesic Effect of Normorphin-3- and --6-Glucuronides." CHEMICAL AND PHARMACEUTICAL BULLETIN, vol. 37, no. 4, 1989, pages 955-957, XP002076325
- S.V.LÖSER ET AL.: "Morphine-6-O-B-D-Glucuronide Binds to u-, d-, and k-specific Opioid Binding Sites in Cerebral Membranes." NAUNYN-SCHMIEDEBERG'S ARCH PHARMACOL., vol. 354, 1996, pages 192-197, XP002076326

## Description

### TECHNICAL FIELD

The present invention relates to morphine derivatives and in particular to a derivative of morphine-6-glucuronide.

### BACKGROUND ART

Morphine and its known derivatives are opiates which have pain relief properties, and are therefore useful in the treatment of chronic and acute pain encountered in various indications in humans and other warm blooded animals. Certain known derivatives may also be used as antidotes in situations of abuse or overdose.

Murphey & Olsen (Journal of Liquid Chromatography (1993), vol. 16, no. 12, 2545-2461), describe a number of examples of morphine derivatives, including hydromorphone and dihydromorphine, and the active morphine metabolites morphine-6-β-D-glucuronide, morphine-3-β-D-glucuronide and normorphine.

Some morphine derivatives are described in PCT applications numbers GB 92/01449 and GB 94/02605, the disclosures of which are incorporated herein by way of reference. These references also disclose a particularly advantageous, new process for the preparation of morphine derivatives which avoids the use of heavy metal salts. A particularly preferred morphine derivative therein is morphine-6-glucuronide.

### DISCLOSURE OF THE INVENTION

It has now been found that a certain morphine-6-glucuronide analogue has particularly useful enhanced agonist opioid activity.

According to the present invention, there is provided a morphine-6-glucuronide analogue having the formula 1-(7,8-dihydromorphine-6-yl)-β-D-glucopyranosiduronic acid (I), and acid addition and metal salts thereof. 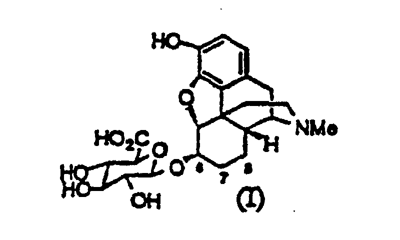 In the morphine structure (II), a number of sites are capable of substitution and modification. For example the ester substituent at position 6 may be pyranose, furanose and uronic acid derivatives thereof; the substituent at the cyclic nitrogen may be for example, hydrogen, lower alkyl (C₁ to C₈), substituted lower alkyl in which the substituents may be aryl, such as for example phenyl, carboxylic acid and esters thereof, and cycloalkyl, for example, cyclopropyl, and N-oxide; the phenolic group may also be alkylated or esterified.

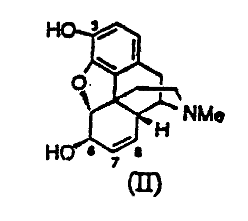

According to the present invention, it has been found that the morphine derivative, wherein the nitrogen substituent is methyl, the ether substituent at position 6 is derived from glucuronic acid, and the ethylenic bond bridging position 7- and 8- is hydrogenated, has enhanced opioid agonist activity compared to morphine in that it has a higher affinity for the opioid analgesia receptor (µ1), and increased anti-nociceptive activity in an animal pain model, as detailed in table 1. Enhanced activity is also attainable when the ethylenic bond bridging position 7- and 8- is modified by other adducts, whereby instead of dihydro, the 7-8- position may be dihydroxy-, hydroxyhalo-, epoxy-, dihalo-, hydrohalo-, hydrohydroxy-, or CXY (X, Y is halogen or hydrogen) adducts.

The compound of the present invention may be in the form of an acid addition salt, such as for example sulphate, hydrochloride, acetate, maleate, tartrate, citrate; and it may be in the form of an alkali metal or alkaline earth salt, such as for example sodium, potassium, lithium, calcium. The compound may also be in the form of a hydrate or solvate.

The present invention includes the compound of the present invention in any crystalline form.

The compound of the present invention may conveniently be prepared by the following route starting from morphine sulphate.
1. Protection of the 3-phenolic group by acylation.
2. Protection of the hydroxyl and carboxylic acid groups of D-glucuronolactone.
3. Condensation of the products of 1 and 2 above.
4. Removal of the protecting groups of the condensation product 3 to yield morphine-6-glucuronide.
5. At any stage as appropriate hydrogenation of morphine-6-glucuronide to yield the product of the present invention.

The compound of the present invention may be administered by any convenient parenteral route. The dose may be varied as required, but will generally, for example, be in the range 0.01 mg/kg to 0.30 mg/kg for patients starting on a strong analgesic, and may be administered continuously, for example, intravenously or subcutaneously.

### DESCRIPTION OF THE PREFERRED EMBODIMENT

The invention is illustrated with reference to the following examples.

### Example 1

*1-(7,8-Dihydromorphin-6-yl)-β-D-glucopyranosiduronic Acid.* Morphine-6-glucuronide (2.00g, 4.02mmol), prepared as described in PCT applications numbers GB 92/01449 and GB 93/02605, was suspended in 1:1 aq. methanol (40ml) and hydrogenated in a Parr apparatus in the presence of 10% Pd-C (0.4g). After 2h, when uptake appeared complete, the mixture was filtered through celite and the precipitate washed well with the same solvent. The combined filtrate and washings were evaporated to a thick gum (2.1g) which was dissolved in warm water (10ml), diluted to 50ml with warm methanol and filtered. After cooling the colourless prisms were filtered off, washed with methanol and dried to give the product (1.86g, 93% in two crops), m. p.>260°C (dec) (Found: C, 55.7; H, 7.3; N 2.5. C₂₃H₂₉NO₉.CH₃OH.H₂O requires C, 56.1; H, 6.8; N 2.7%); δ (D₂O), *inter alia*, 0.90 (1 H, s), 1.83 (1 H, m), 2.05, (1 H, dt), 2.35 (1 H, m), 2.82 (3 H, s), 3.57 (1 H, d), 3.80 (1 H, m), 4.12 (1 H, m), 4.47 (1 H, d), 4.86 (1 H, d), 6.65 and 6.74 (2 H, 2 d); m/z (E.l 463 (M⁺). HPLC area purity 99.6%.

The compound of the invention was compared' with some other similar morphine analogues.

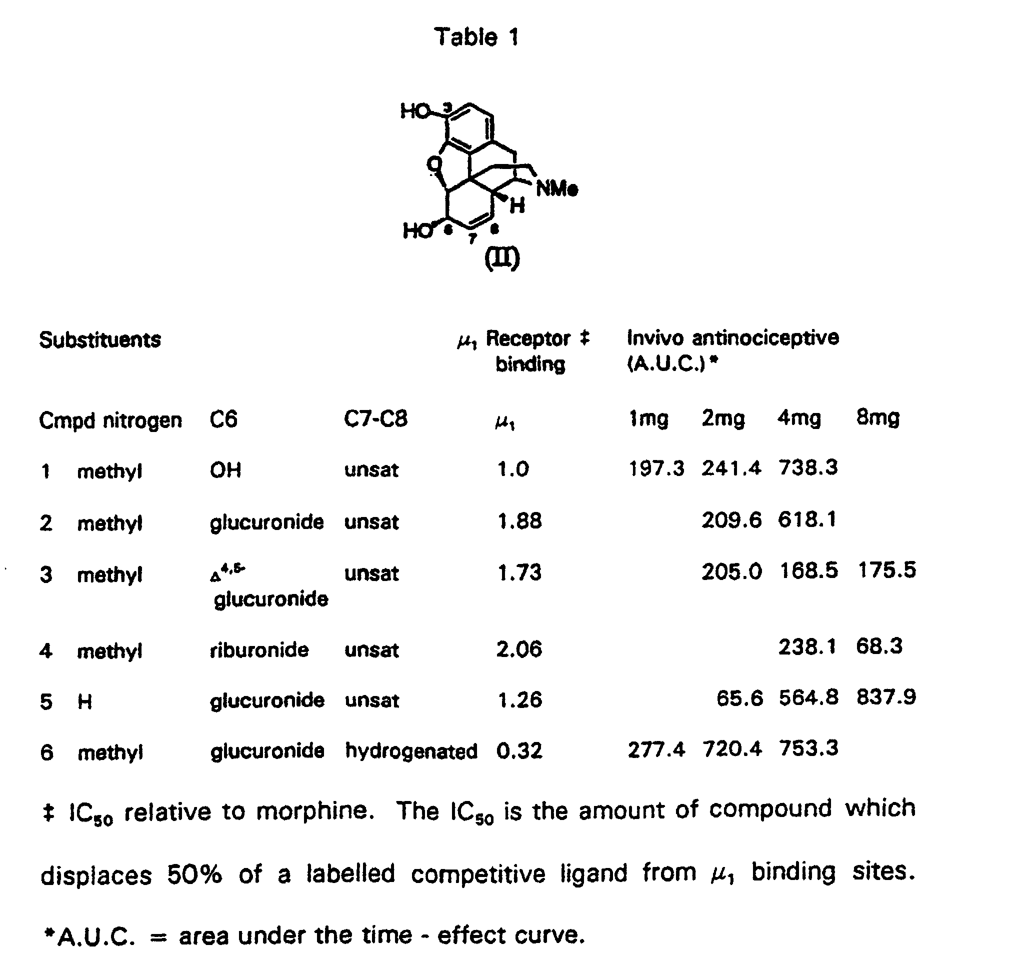

In Table 1, compound 1 is morphine, compound 2 is morphine-6-glucuronide, and compound 6 is the compound of the present invention. Compounds 3 and 4 were prepared by a method analogous to that described above for compound 6; compound 5 required removal of the N-methyl group prior to hydrolysis of the protecting groups.

The results in Table 1 show that the compound of the present invention has the greatest binding affinity to the relevant receptor (µ₁) (lowest lC₅₀ value relative to morphine), and has the greatest potency in an in-vivo model system of antinociceptive activity.

## Claims

1. A compound of the following formula (I), or a pharmaceutically acceptable salt thereof: 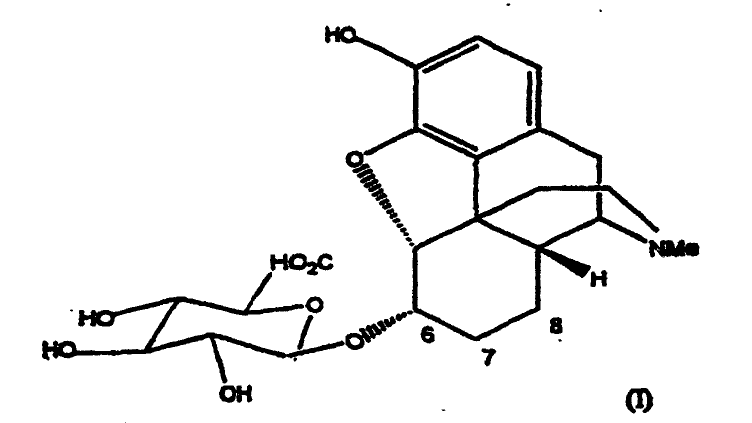 wherein
the 7-8- position is dihydro-, dihydroxy-, hydroxyhalo-, epoxy-, dihalo-, hydrohalo-, hydrohydroxy-, or CXY (X, Y is halogen or hydrogen) adducts.

2. A compound as claimed in claim 1, wherein the 7-8- position is dihydro.

3. A pharmaceutical composition comprising a pharmaceutically effective amount of a compound of formula (I) as defined in claim 1, or a pharmaceutically acceptable salt thereof.

4. A compound of formula (I) as defined in claim 1, or a pharmaceutically acceptable salt thereof, for use as a medicament for the treatment of chronic or acute pain.

5. A compound of formula (I) as defined in claim 1, or a pharmaceutically acceptable salt thereof, for use as a medicament for the treatment of a morphine overdose.

6. Use of a compound of formula (l) as defined in claim 1, or a pharmaceutically acceptable salt thereof, for the manufacture of a medicament for the treatment of chronic or acute pain.

7. Use of a compound of formula (I) as defined in claim 1, or a pharmaceutically acceptable salt thereof, for the manufacture of a medicament for the treatment of a morphine overdose.

8. A process for making a compound of the formula: 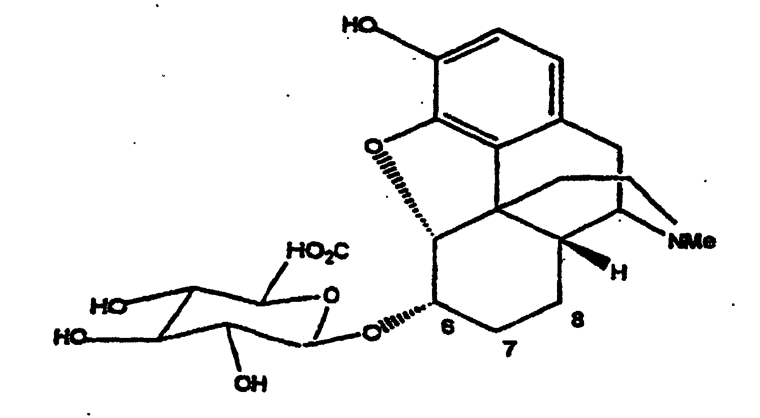 comprising:
(a) protecting the 3-phenolic group of morphine sulphate by acylation,
(b) protecting the hydroxyl and carboxylic acid groups of D-glucuronolactone.
(c) condensing the products of steps (a) and (b),
(d) removing the protecting groups of the condensation product obtained in step (c) to yield morphine-6-glucuronide, and
(e) hydrogenating the morphine-6-glucuronide.

## Patentansprüche

1. Verbindung der folgenden Formel (I) oder pharmazeutisch akzeptables Salz hiervon: 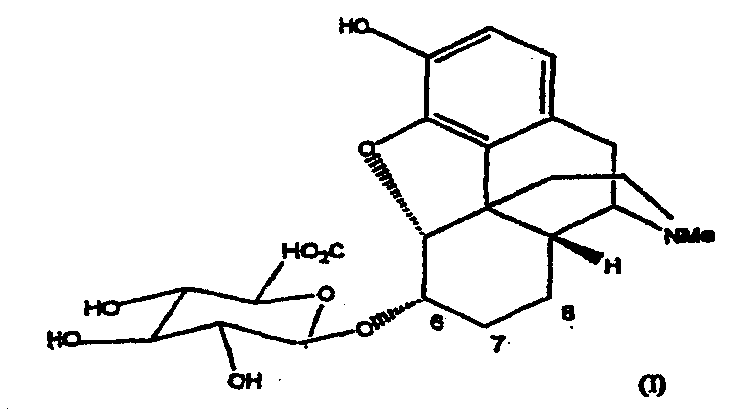 wobei die 7-8-Position Dihydro-, Dihydroxy-, Hydroxyhalo-, Epoxy-, Dihalo-, Hydrohalo-, Hydrohydroxy- oder CXY(X, Y ist Halogen oder Wasserstoff)-Addukte aufweist.

2. Verbindung nach Anspruch 1, wobei die 7-8-Position dihydro ist.

3. Pharmazeutische Zusammensetzung, die eine pharmazeutisch wirksame Menge einer Verbindung der Formel (I) nach Anspruch 1 oder ein pharmazeutisch akzeptables Salz davon aufweist.

4. Verbindung der Formel (I) nach Anspruch 1 oder ein pharmazeutisch akzeptables Salz davon zur Verwendung als Medikament für die Behandlung von chronischen oder akuten Schmerzen.

5. Verbindung der Formel (I) nach Anspruch 1 oder ein pharmazeutisch akzeptables Salz davon zur Verwendung als Medikament zur Behandlung einer Morphin-Überdosis.

6. Verwendung einer Verbindung der Formel (I) nach Anspruch 1 oder eines pharmazeutisch akzeptablen Salzes davon zur Herstellung eines Medikamentes für die Behandlung chronischer oder akuter Schmerzen.

7. Verwendung einer Verbindung der Formel (I) nach Anspruch 1 oder eines pharmazeutisch akzeptablen Salzes davon, zur Herstellung eines Medikamentes zur Behandlung einer Morphin-Überdosis.

8. Verfahren zur Herstellung einer Verbindung der Formel: 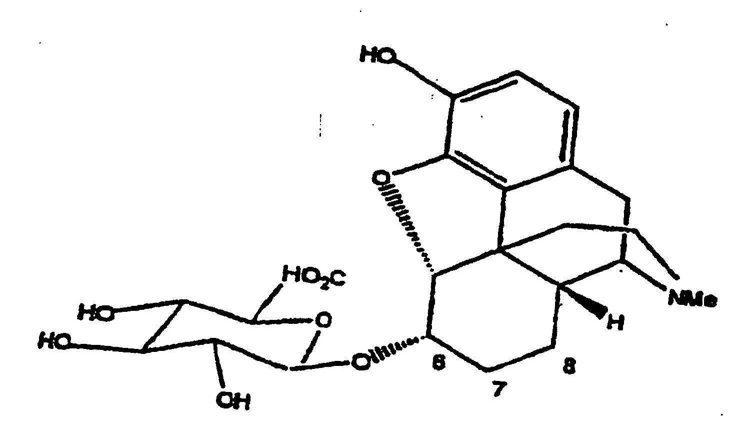 das aufweist:
(a) Schützen der 3-Phenolgruppe von Morphinsulfat durch Acylierung,
(b) Schützen der Hydroxyl- und der Carboxysäure-Gruppen von D-Glucuronolacton.
(c) Kondensieren der Produkte der Schritte (a) und (b),
(d) Entfernen der Schutzgruppen des im Schritt (c) erhaltenen Kondensationsproduktes, um Morphin-6-Glucuronid zu erhalten, und
(e) Hydrieren des Morphin-6-Glucuronids.

## Revendications

1. Composé de la formule suivante (I), ou un sel pharmaceutiquement acceptable de celui-ci : 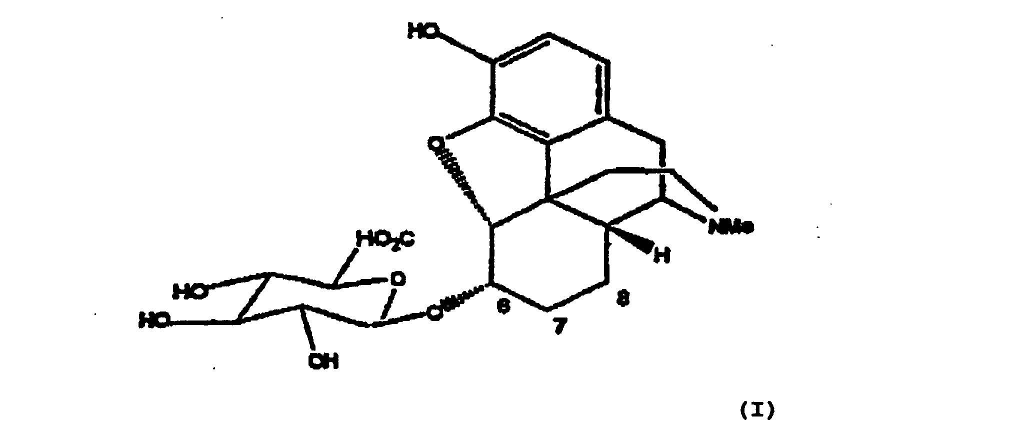 dans laquelle
la position 7-8 est un adduit dihydro-, dihydroxy-, hydroxyhalo-, époxy-, dihalo-, hydrohalo-, hydrohydroxy-, ou CXY (X, Y est un halogène ou un hydrogène).

2. Composé selon la revendication 1, dans lequel la position 7-8 est dihydro.

3. Composition pharmaceutique comprenant une quantité pharmaceutiquement efficace d'un composé de formule (I) selon la revendication 1, ou d'un sel pharmaceutiquement acceptable de celui-ci.

4. Composé de formule (I) selon la revendication 1, ou un sel pharmaceutiquement acceptable de celui-ci, pour l'utilisation comme médicament pour le traitement de douleur chronique ou aiguë.

5. Composé de formule (I) selon la revendicationl, ou un sel pharmaceutiquement acceptable de celui-ci, pour l'utilisation comme médicament pour le traitement d'une overdose de morphine.

6. Utilisation d'un composé de formule (I) selon la revendication 1, ou d'un sel pharmaceutiquement acceptable de celui-ci, pour la préparation d'un médicament pour le traitement de douleur chronique ou aiguë.

7. Utilisation d'un composé de formule (I) selon la revendication 1, ou d'un sel pharmaceutiquement acceptable de celui-ci, pour la préparation d'un médicament pour le traitement d'une overdose de morphine.

8. Procédé pour préparer un composé de formule : 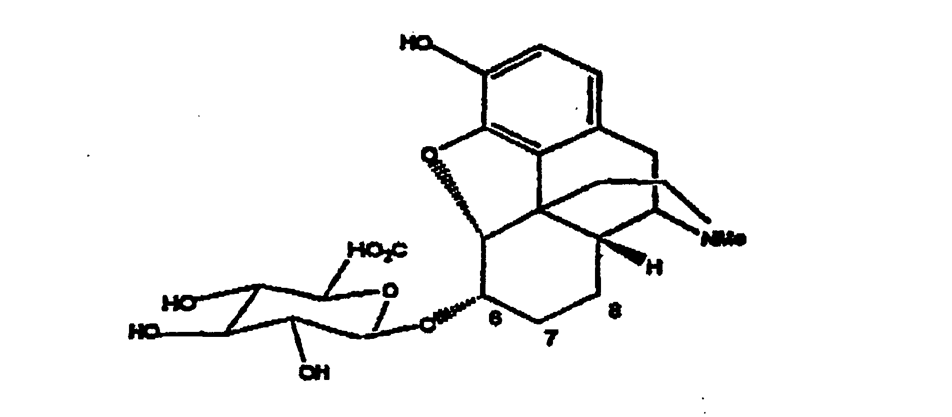 comprenant :
(a) la protection du groupe 3-phénolique du sulfate de morphine par acylation.
(b) la protection des groupes hydroxyle et acide carboxylique de la D-glucurolactone,
(c) la condensation des produits des étapes (a) et (b),
(d) l'élimination des groupes protecteurs du produit de condensation obtenu dans l'étape (c) pour conduire à la morphine-6-glucuronide, et
(e) l'hydrogénation de la morphine-6-glucuronide.
